# EUROPÄISCHE PATENTSCHRIFT

(11) **EP 2 070 528 B1**
(45) Veröffentlichungstag und Bekanntmachung des Hinweises auf die Patenterteilung: **02.07.2014**
(21) Anmeldenummer: 07024212.8
(22) Anmeldetag: 13.12.2007
(51) Int. Cl.: A61K 8/44, A61Q 11/00, A61K 31/198, A61Q 15/00, A61P 1/00

(54) **Verfahren zur Geruchsextinktion**
Method for absorbing odours
Procédé destiné à l'élimination d'odeurs

(43) Veröffentlichungstag der Anmeldung: 17.06.2009
(73) Patentinhaber: Pieper, Gudrun, 48159 Münster (DE); Husmann, Karl-Heinz, 48159 Münster (DE)
(72) Erfinder: Pieper, Gudrun, 48159 Münster (DE); Husmann, Karl-Heinz, 48159 Münster (DE)
(74) Vertreter: Jostarndt, Hans-Dieter

(56) Entgegenhaltungen:
- EP-A- 1 195 099
- WO-A-00/23040
- WO-A-2007/016644
- DE-A1- 19 854 987
- US-B1- 6 403 642

## Beschreibung

Alliumgewächse sind als Gewürze oder Beilagen in einer Vielzahl von Speisen und Zubereitungen welt verbreitet. Dies gilt insbesondere für Knoblauch (*Allium sativum*), Zwiebeln (*Allium cepa*), Schalotten (*Allium ascalonium*) sowie wie auch Bärlauch *(Allium ursinum)* oder Schnittlauch *(Allium schoenoprasum*). Diese Pflanzen gehören jeweils der Gattung Allium und der Familie der Zwiebelgewächse (*Alliaceae*) an. Neben der rein geschmacklichen Komponente der Alliumgewächse bzw. deren Teile, die zu ihrem Verzehr veranlassen, stehen bei ihrer Nutzung auch gesundheitliche Aspekte im Vordergrund. So ist nämlich beispielsweise von Knoblauch bekannt, dass dessen Inhaltsstoffe antimikrobiell wirken. Außerdem gibt es viele Hinweise darauf, dass dessen Verzehr zu einer Senkung von Blutfettwerten führt und somit vorbeugend gegen arterlosklerotische Veränderungen der Blutgefäße wirkt. Auch kann sich die Viskosität des Blutes verändern, was beispielsweise in *In-vitro* Tests an humanen Blutplättchen nachgewiesen wurde, die mit Knoblauchextrakten behandelt wurden.

Auch für die Zwiebel (*Allium cepa*) sind verschiedene medizinische Verwendungen bekannt. So gilt der Saft frischer Zwiebeln als altes Hausmittel gegen Erkältungskrankheiten und Husten. Darüber hinaus werden jedoch auch - ähnlich wie bei Knoblauch - antimikrobielle, blutfettwertsenkende sowie blutgerinnungshemmende und blutzuckersenkende Eigenschaften diskutiert. Demnach kann der Verzehr von Zwiebeln positive Auswirkungen auf das gesamte Herzkreislaufsystem haben und ebenso der Vorbeugung arteriosklerotischer Veränderungen dienen.

Ein ähnliches Wirkspektrum wird gleichermaßen dem Schnittlauch und dem Bärlauch zugesprochen. Auch hier wird von bakterienhemmenden, leicht blutdrucksenkenden sowie der Blutaggregierung vorbeugenden Wirkung und einer Senkung des Cholesterinspiegels berichtet.

Es lässt sich somit zusammenfassen, dass eine Vielzahl von Vertretern der Alliumgewächse sowohl aus geschmacklichen Gründen als auch aus medizinischen Gründen bereits seit vielen Jahrhunderten eine weite Verbreitung gefunden hat.

In der Zwischenzeit weiß man, dass für die verschiedenartigen pharmakologischen Effekte der Alliumgewächse vor allem schwefelhaltige sekundäre Pflanzeninhaltsstoffe verantwortlich sind. Ein besonders wichtiger Vertreter davon ist das Alliln ((+)-(S)-Allyl-L-cysteinsulfoxid). Diese Verbindung ist zunächst geruchlos und vermutlich pharmakologisch unwirksam. Erst durch eine Verletzung des pflanzlichen Gewebes kommt das Alliin in Kontakt mit der Alliinase und wird in einer katalytischen Reaktion in das Allicin (2-Propen-Thiosulfinsäure-(S)-Allylester) umgewandelt, was dann für den von Knoblauch oder anderen Alliumgewächsen bekannten typischen Geruch sorgt. Ebenso geht die pharmakologische Wirksamkeit der Alliumgewächse nach derzeitigem Kenntnisstand zu großen Teilen auf das Allicin zurück, das nämlich antibakteriell, antimykotisch und antibiotisch wirkt.

Allicin ist hoch reaktiv und ist somit Ausgangsprodukt einer Vielzahl von des weiteren in Alliumgewächsen auftretenden schwefelhaltigen Verbindungen. Dazu gehören beispielsweise das Thioacrolein, das Cycloalliin oder Dialkylsulfide, Thiosulfonate oder S-Oxide. Das Ajoen, das nach einer beta-Eliminierung gemeinsam mit dem Thioacrolein aus dem Allicin hervorgeht, wird beispielsweise für die antithrombotische Wirkung von Knoblauchextrakten verantwortlich gemacht. Ebenso löst es bei menschlichen Leukämiezellen eine Apoptose aus, und hat daher eine potentiell anticancerogene Wirkung.

Der Verzehr von Alliumgewächsen ist jedoch mit dem erheblichen Nachteil verbunden, dass das bei der mechanischen Zerstörung oder dem Aufschluß der pflanzlichen Gewebe durch die Katalyse von Alliin entstehende Allicin stark geruchsbildend ist. Diese Geruchsbildung wird von der überwiegenden Mehrzahl der Menschen als äußerst unangenehm und belästlgend empfunden. Somit geht mit dem aus geschmacklichen und medizinischen Gründen vorteilhaften Verzehr von Alliumgewächsen stets der Nachteil einher, die Menschen der unmittelbaren Umgebung durch die damit verbundene Geruchsbildung in Mitleidenschaft zu ziehen. Dies sollte jedoch gerade im privaten sowie auch im beruflichen Kontext möglichst vermieden werden.

Es sind Verfahren nach dem Stand der Technik bekannt, die zu einer Verminderung der Geruchsbildung nach dem Verzehr von Alliumgewächsen führen sollen. So wird beispielsweise empfohlen, die unangenehme Geruchsbildung durch die Zubereitung der Speise zu reduzieren, indem die Hände nach der Zerkleinerung von Zwiebeln oder Knoblauch mit Zitronensaft gewaschen werden. Alternativ wird die Verwendung von "Metallseifen" für den Abbau des Geruchs vorgeschlagen.

US 6403642 beschreibt Metallkomplexe von Cystein zur Verminderung von Atemgerüchen verursacht durch schwefeilhaltige Verbindungen.

Diese Maßnahmen sind jedoch auf den Bereich der Hände reduziert. Da der durch Allicin oder andere Disulfidbrücken haltigen Verbindungen verursachte typische "Knoblauch"- oder "Zwiebelgeruch" nach dem Verzehr alliumhaltiger Speise aber nicht auf die Hände beschränkt ist, sondern vor allem auch den Atem betrifft, sind diese Maßnahmen in ihrer Wirkung nur unzureichend.

Es ist somit Aufgabe der vorliegenden Erfindung, ein Verfahren und ein Mittel zur Verfügung zu stellen, was die mit dem Verzehr alliumhaltiger Speisen verbundene Geruchsbildung reduziert bzw. den Geruch selber vermeidet oder möglichst abbaut.

Diese Aufgabe wird gelöst durch die Verwendung von Acetylcystein zur Behandlung der Geruchsbildung aufgrund des Verzehrs alliumhaltiger Gewächse oder Teilen davon in Menschen. Darüber hinaus wird ein Mittel bzw. eine Zubereitung vorgeschlagen, das bzw. die diesem Zwecke dient.

Im Rahmen der vorliegenden Erfindung wird unter dem Terminus "Behandlung" jede Maßnahme verstanden, die zu der Vermeidung, der Linderung oder zu einem Abbau (Extinktion) der Geruchsbildung bzw. des Geruchs im oder am menschlichen Körper führt. Es ist somit Gegenstand der Erfindung, das erfindungsgemäße Mittel entweder vor, während oder auch nach dem Verzehr der Alliumgewächse einzunehmen oder zu verabreichen bzw. die erfindungsgemäßen Maßnahmen zu ergreifen. Die Erfindung kann somit sowohl prophylaktisch als auch zur Verminderung einer ggf. fortschreitenden Geruchsbildung sowie auch zur Verringerung eines bereits bestehenden Geruchs (Geruchsextinktion) eingesetzt werden.

Die erfindungsgemäße Wirkung beruht im wesentlichen darauf, dass Disulfidbrücken innerhalb der geruchsbildenden schwefelhaltigen Pflanzeninhaltsstoffe, wie z. B. des Allicins, gespalten werden. Diese Spaltung kann entweder oxidativ oder durch eine Reduktion erfolgen.

Erfindungsgemäß einsetzbares Mittel zur Spaltung von Disulfidbrücken ist das Acetylcystein als Mukolytikum. Das Acetylcystein als pharmazeutisch akzeptables Mukolytikum ist in seiner Anwendung als Expektorans, d. h. als schleimlösende Substanz, bekannt.

Es wurde bereits dargelegt, dass das erfindungsgemäße Verfahren der Geruchsbildung durch den Verzehr von Alliumgewächsen bzw. Teilen entgegenwirkt bzw. den Geruch abbaut. Im Sinne der vorliegenden Erfindung wird der Begriff "Alliumgewächs bzw. Teile daraus" umfassend verstanden, d. h. jeder Bestandteil pflanzlichen Ursprungs, demnach alle unterirdischen oder oberirdischen Teile einschließlich alle Bereiche der Wurzeln, der Blätter, der Früchte oder der Samen sowie der Blüten sind von dieser Definition erfaßt.

Das erfindungsgemäß einsetzbare Acetylcystein kann in verschiedenen dem Fachmann geläufigen Darreichungsformen verabreicht oder eingenommen werden, so z. B. als Kapsel, Tablette, beispielsweise Brausetablette, Saft, Pulver oder als Inhalationsmittel. Auch Injektionslösungen sind möglich. Vorteilhafterweise liegt die pro Anwendung verabreichte Menge an Acetylcystein unterhalb derjenigen Dosierung, die für eine bekannte pharmakologische Wirkung notwendig ist.

Die für die pharmakologische Wirkung des Acetylcysteins als Schleimverflüssiger üblicherweise verabreichte Tagesdosis in peroraler Darreichungsform liegt bei 600 mg für Erwachsene (ggf. 2-3 mal täglich 200 mg), bei Kindern von 6 bis 14 Jahren bei 300 mg/d (oder ein 1-3 mal täglich 100 mg) sowie bei Kleinkindern unter 2 Jahren bei 2-3 mal täglich je 50 mg. Erfindungsgemäß kann die für die beobachtete Geruchsextinktion oder Vermeidung der Geruchsbildung bei Erwachsenen erforderliche Einmaldosis bei 100 mg oder weniger pro Anwendung liegen. Vorteilhafterweise liegt sie sogar bei bis zu 75 mg, insbesondere bevorzugt bis zu 50 mg oder bis zu 25 mg, pro Anwendung bei Erwachsenen. Die Dosis kann auch bei etwa 40 mg liegen. Erforderlichenfalls kann die Menge mehrfach gewonnen werden. Erfahrungsgemäß kann somit die Dosierung von 0,01 mg/kg Körpergewicht bis 2,5 mg/kg, bevorzugt von 0,05 bis 2,0 mg/kg Körpergewicht verabreicht werden.

Die oben genannten Dosierungen können Gegenstand einer entsprechenden Dosiseinheltsform sein (beispielsweise einer Tablette, eines Sachets, einer Kapsel oder einer definierten Spraymenge bei Inhalationsgeräten).

Die erfindungsgemäßen Zusammensetzungen oder Zubereitung können die für beispielsweise Acetylcysteinformulierungen üblichen Hilfs- und Trägerstoffe umfassen, beispielsweise Hilfsstoffe aus der Gruppe folgender Substanzen: Aspartam, Zitronensäure, Natriumbicarbonat, Natrium, Phenylallanin oder Zitronenaroma. Dies gilt beispielsweise für die Formulierung als Brausetabletten. Sofern das Acetylcystein als Pulver dargereicht wird, kann es beispielsweise Ascorbinsäure (Vitamin C), Saccharin, Sucrose oder Aromastoffe enthalten. Weitere Formulierungsbeispiele enthalten beispielsweise zusätzlich Lactose, Manitol, Natrium in Form von Natriumcitrat, Natriumhydrogencarbonat, Natriumcarbonat und Natriumcyclamat. Ebenso können Saccharin-Natrium und Aromastoffe hinzugefügt werden.

Die Erfindung betrifft des weiteren verkaufsfertige Verpackungseinheiten enthaltend Zubereltungen mit dem erfindungsgemäß einsetzbaren Acetylcystein als Nahrungsergänzungsmittel oder Life-Style Produkt.

### Beispiele:

1. Verabreichung von Acetylcystein nach dem Verzehr einer knoblauchhaltigen Mahlzeit.
Testreihe mit 15 Tests pro Person.

| | |
|---|---|
| Dosierung: | 50 mg bei weiblicher Testperson (32 bis 74 kg Körpergewicht) |
| | 75 mg bei männlicher Testperson (52 bis 98 kg Körpergewicht) |
| Ergebnis: | Geruchslosigkeit in allen Tests |

Für diese Tests wurden ACC-Brausetabletten der Firma Hexal verwendet.
Das positive Testergebnis stellte sich jeweils in einem Zeitfenster von 10 bis 20 Minuten nach Verabreichung des Acetylcysteins ein.
2. Verabreichung das Acetylcyateins vor dem Verzehr der knoblauchhaltigen Mahlzeit
Testreiche und Dosierung wie unter Ziffer 1. ausgeführt.
Die Verabreichung des Acetylcysteins erfolgte etwa 10 Minuten vor der Mahlzeit. Auch in diesen Tests wurde die Geruchsbildung verhindert.

## Patentansprüche

1. Verwendung eines pharmazeutisch akzeptablen Reduktionsmittels mit reaktiver Thiolgruppe für Disulfidbrücken-haltige organische Verbindungen zur Behandlung von Geruchsbildung im Menschen aufgrund des Verzehrs von Alliumpflanzen oder Teilen davon, **dadurch gekennzeichnet, dass** als Reduktionsmittel Acetylcystein eingesetzt wird.

2. Verwendung nach Anspruch 1, **dadurch gekennzeichnet, dass** Acetylcystein vor dem Verzehr, vorzugsweise innerhalb einer Stunde vor dem Verzehr, verabreicht wird.

3. Verwendung nach Anspruch 1, **dadurch gekennzeichnet, dass** Acetylcystein nach dem Verzehr des Alliumgewächses verabreicht wird.

4. Verwendung nach einem der vorherigen Ansprüche, **dadurch gekennzeichnet, dass** es sich bei dem Alliumgewächs um eines aus der Gruppe der folgenden Gewächse handelt: *Allium sativum (Knoblauch), Allium cepa (Zwiebel), Allium ursinum (Bärlauch) oder Allium schoenoprasum (Schnittlauch).*

5. Verwendung nach einem der vorherigen Ansprüche, **dadurch gekennzeichnet, dass** die verabreichte Tagesdosis In peroraler Form für das Acetylcystein bei 600 mg für Erwachsene, bei 300 mg für Kinder von 6 bis 14 Jahren, und bei 150 mg für Kleinkinder unter 2 Jahren liegt.

6. Verwendung nach einem der vorherigen Ansprüche, **dadurch gekennzeichnet, dass** die Dosierung des Acetylcysteins zwischen 25-125 mg vorzugsweise zwischen 40 und 100 mg, besonders bevorzugt zwischen 50 und 75 mg pro Anwendung beträgt.

7. Verwendung von Acetylcystein zur Herstellung eines Mittels zur Behandlung von Geruchsbildung im Menschen aufgrund des Verzehrs von Alliumpflanzen oder Teilen davon In einer Dosiseinheltsform, die eine Verabreichung von 0,01 bis 2,5 mg/kg Körpergewicht des Patienten erlaubt.

## Claims

1. The use of a pharmaceutically acceptable reducing agent having a reactive thiol group for organic compounds containing disulfide bridges for the treatment of odor formation in humans due to the consumption of plants of the *Allium* species or parts thereof, **characterized in that** acetylcysteine is employed as the reducing agent.

2. The use according to claim 1, **characterized in that** acetylcysteine is administered before consumption, preferably within one hour prior to consumption.

3. The use according to claim 1, **characterized in that** acetylcysteine is administered after the consumption of the *Allium* plant.

4. The use according to one of the preceding claims, **characterized in that** the *Allium* plant belongs to the following group of plants: *Allium sativum* (garlic), *Allium cepa* (onion), *Allium ursinum* (wild garlic) or *Allium schoenoprasum* (chives).

5. The use according to one of the preceding claims, **characterized in that** the administered daily dose of acetylcysteine in peroral form is 600 mg for adults, 300 mg for children between the ages of 6 and 14, and 150 mg for small children who are less than 2 years old.

6. The use according to one of the preceding claims, **characterized in that** the dosage of acetylcysteine is between 25 mg and 125 mg, preferably between 40 mg and 100 mg, especially preferably between 50 mg and 75 mg per application.

7. The use of acetylcysteine to produce an agent for the treatment of odor formation in humans due to the consumption of plants of the *Allium* species or parts thereof, in the form of a dose unit allowing the administration of 0.01 to 2.5 mg/kg of body weight of the patient.

## Revendications

1. Utilisation d'un agent de réduction pharmaceutiquement acceptable comprenant un groupe thiol réactif pour composés organiques contenant des ponts disulfure pour le traitement de l'apparition d'odeurs chez l'humain en raison de la consommation de plantes d'Allium ou de parties de ces plantes, **caractérisée en ce que** l'acétylcystéine est utilisée en tant qu'agent de réduction.

2. Utilisation selon la revendication 1, **caractérisée en ce que** l'acétylcystéine est administrée avant la consommation, de préférence dans l'heure précédant la consommation.

3. Utilisation selon la revendication 1, **caractérisée en ce que** l'acétylcystéine est administrée après la consommation de la plante d'Allium.

4. Utilisation selon l'une des revendications précédentes, **caractérisée en ce que** la plante d'Allium est une plante du groupe des plantes suivantes : Allium sativum (ail cultivé), Allium cepa (oignon), Allium ursinum (ail sauvage) ou Allium schoenoprasum (ciboulette).

5. Utilisation selon l'une des revendications précédentes, **caractérisée en ce que** la dose quotidienne d'acétylcystéine administrée par voie orale est de 600 mg pour les adultes, de 300 mg pour les enfants de 6 à 14 ans et de 150 mg pour les jeunes enfants de moins de 2 ans.

6. Utilisation selon l'une des revendications précédentes, **caractérisée en ce que** la dose d'acétylcystéine est comprise entre 25 et 125 mg, de préférence entre 40 et 100 mg et de manière particulièrement préférentielle entre 50 et 75 mg par application.

7. Utilisation d'acétylcystéine pour fabriquer un agent permettant de traiter l'apparition d'odeurs chez l'humain due à la consommation de plantes d'allium ou de parties de ces plantes, sous la forme de doses unitaires permettant d'administrer de 0,01 à 2,5 mg/kg de poids corporel du patient.
